# EUROPEAN PATENT SPECIFICATION

(11) **EP 4 003 957 B1**
(45) Date of publication and mention of the grant of the patent: **17.04.2024**
(21) Application number: 20746194.8
(22) Date of filing: 24.07.2020
(51) Int. Cl.: C07D 213/74

(54) **PROCESS FOR THE SYNTHESIS OF N-ALKYL-4-PYRIDINAMINES**
VERFAHREN ZUR SYNTHESE VON N-ALKYL-4-PYRIDINAMINEN
PROCÉDÉ DE SYNTHÈSE DE N-ALKYL-4-PYRIDINAMINES

(30) Priority: 25.07.2019 EP 19382636; 13.11.2019 EP 19382999
(43) Date of publication of application: 01.06.2022
(73) Proprietor: Medichem, S.A., 08970 Sant Joan Despi, Barcelona (ES)
(72) Inventor: CONTRERAS LASCORZ, Juan, 17460 CELRÀ (ES); DURÁN LÓPEZ, Ernesto, 08755 CASTELLBISBAL (ES)
(74) Representative: ZBM Patents - Zea, Barlocci & Markvardsen
(86) International application number: PCT/EP2020/070980
(87) International publication number: WO 2021/013997

(56) References cited:
- WO-A1-95/21151
- WO-A2-2009/136409
- US-A- 4 206 215

## Description

This application claims the benefit of European Patent Application EP19382636.9 filed July 25th, 2019 and European Patent Application EP19382999.1 filed November 13th, 2019.

### FIELD OF THE INVENTION

The present invention relates to an improved process for the synthesis of *N*-alkyl-4-pyridinamines.

### BACKGROUND OF THE INVENTION

*N*-alkyl-4-pyridinamines of Formula I, 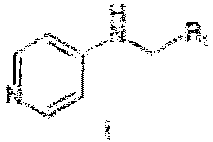 wherein R₁ is a hydrogen or an alkyl having 1 to 11 carbon atoms,
are intermediates useful in the preparation of bis(N-alkylpyridin-4(1H)-imines and their salts.

It is known that the reaction of 4-aminopyridine with an aldehyde compound in a solvent results in the formation of an imine, also known as Schiff base which is a chemical compound containing a carbon-nitrogen double bond. The hydrogenation of the obtained imine or Schiff base results in the formation of the corresponding amine. The overall process is known as reductive amination.

US4206215 discloses the preparation of *N*-heptyl-4-pyridinamine, *N*-octyl-4-pyridinamine and *N*-nonyl-4-pyridinamine which are compounds of formula I wherein R₁ is *n*-hexyl, *n*-heptyl and *n*-octyl, respectively. These compounds are obtained by hydrogenating the corresponding imine compound of formula II wherein R₁ is *n*-hexyl, *n-*heptyl and *n*-octyl, respectively, under hydrogen pressure in the presence of ethanol and palladium-on-carbon (Pd/C) catalyst.

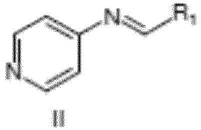

Although it is not specifically disclosed in US4206215, the imine compounds of formula II wherein R₁ is *n*-hexyl, *n*-heptyl and *n*-octyl are obtained when 4-aminopyridine interacts with the aldehyde compound containing the appropriate carbon content (heptanal, octanal and nonanal, respectively). The reaction takes place using a 3:1 molar ratio of aldehyde compound with respect to 4-aminopyridine, and the hydrogenation is performed using 7.4% w/w of Pd/C catalyst with respect to 4-aminopyridine. No purities are provided for the final product.

RU2345068C2 discloses the preparation of *N*-octyl-4-pyridinamine (compound of formula I wherein R₁ is *n*-heptyl) by hydrogenating the imine compound of formula II wherein R₁ is *n*-heptyl in the presence of a C₃-C₄-alcohol and Pd/C catalyst.

Although it is not specifically disclosed in RU2345068C2, the imine compound of formula II wherein R₁ is *n*-heptyl is obtained when 4-aminopyridine interacts with octanal. The reaction takes place using a 1.5:1 to 2:1 molar ratio of octanal with respect to 4-aminopyridine, and both reagents are contacted at 80-90 °C for 0.8 to 1.2 hours to form the corresponding imine compound of formula II wherein R₁ is *n*-heptyl. Subsequently, this imine compound is hydrogenated using 8-10% w/w of Pd/C catalyst with respect to 4-aminopyridine. No purities are provided for the final product.

The authors of the present invention have found that the processes of the prior art lead to a *N*-alkyl-4-pyridinamine of formula I with undesirable impurities. This results in an inefficient process which also affects the chemical purity of the final bis(N-alkylpyridin-4(1H)-imine. Moreover, the processes of the prior art use higher amounts of catalyst, which is undesirable from an environmental and economical perspective.

Accordingly, there is an unmet need for an efficient catalytic hydrogenation process to produce *N*-alkyl-4-pyridinamines. The present invention provides such improved process.

### BRIEF SUMMARY OF THE INVENTION

It is an object of the present invention to provide an efficient catalytic hydrogenation process to produce *N*-alkyl-4-pyridinamines on a large, commercial scale which overcomes the drawbacks of the processes disclosed in the prior art.

### BRIEF DESCRIPTION OF THE DRAWINGS

Figure 1 shows the X-Ray Powder Diffractogram of *N*-octylpyridin-4-amine prepared as in Example 3.
Figure 2 shows the X-Ray Powder Diffractogram of octenidine dihydrochloride prepared as in Example 4.
Figure 3 shows the X-Ray Powder Diffractogram of octenidine dihydrochloride prepared as in Example 5.
Figure 4 shows the X-Ray Powder Diffractogram of octenidine base prepared as in Example 6.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims.

A first aspect of the present invention provides a process for preparing a *N*-alkyl-4-pyridinamine of formula I, 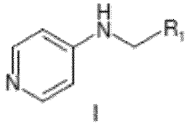 wherein R₁ is hydrogen or an alkyl having 1 to 11 carbon atoms,
which comprises the catalytic hydrogenation of the compound of formula II 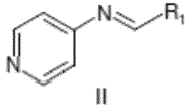 characterized in that the hydrogenation process is carried out in presence of an acid, a palladium catalyst, and an alcoholic solvent.

The authors of the present invention have found that the use of an acid in the hydrogenation process surprisingly increases the purity of the *N*-alkyl-4-pyridinamine of formula I obtained. The process allows to obtain a *N*-alkyl-4-pyridinamine of formula I with a purity ≥99.5%, in particular, a *N*-alkyl-4-pyridinamine of formula I with R₁ = *n-*heptyl. Namely, the process allows to obtain a *N*-alkyl-4-pyridinamine of formula I having less than 0.05% (w/w) of 4-aminopyridine as detected by HPLC and less than 0.15% of the corresponding Schiff base (compound of formula II), in particular, a *N*-alkyl-4-pyridinamine of formula I with R₁ = *n*-heptyl.

The term "alkyl" refers to a straight-chain or branched-chain alkyl group having 1 to 11 carbon atoms, preferably represents a straight-chain.

In a preferred embodiment of the present invention, the alkyl is selected from the group consisting of methyl, ethyl, and linear or branched propyl, butyl, pentyl, hexyl, heptyl, octyl, nonyl, decyl and undecyl.

In a preferred embodiment of the present invention, the alkyl is *n*-heptyl.

The term "catalytic hydrogenation" refers to the treatment with hydrogen in the presence of a catalyst.

The term "catalyst" refers to
palladium catalyst.

In a preferred embodiment of the present invention, the catalyst is palladium supported on activated carbon (palladium-on-carbon, Pd/C).

Preferably, the amount of catalyst used is from 2.5% w/w to less or equal than 7.4% w/w, more preferably 3.5% w/w, in relation to the amount of 4-aminopyridine that has been used in the preparation of the compound of formula II.

Preferably, the catalytic hydrogenation is carried out under a pressure from about atmospheric up to 6 bar. Preferred specific pressure ranges are from about 4.0 to 5.0 bar.

Preferably, the catalytic hydrogenation takes place at a temperature from about 60 °C to about 95 °C, more preferably from about 70 °C to about 85 °C, even more preferably at about 80 °C.

The term "about" or "around" as used herein refers to a range of values ± 10% of a specified value. For example, the expression "about 10" or "around 10" includes ± 10% of 10, i.e. from 9 to 11.

A hydrogenation process in general takes place in the presence of an organic solvent. Non-limiting examples of organic solvents, which can be used alone or as a mixture of solvents, include polar aprotic solvents such as dimethylsulfoxide, *N,N-*dimethylformamide, *N*,*N*-dimethylacetamide, *N*,*N*-dimethylformamide and the like; esters solvents such as butyl acetate, ethyl acetate, ethyl formate, isobutyl acetate, isopropyl acetate, methyl acetate, methyl formate, propyl acetate and the like; alcohol solvents such as methanol, ethanol, *n*-propanol, isopropanol, *n*-butanol and the like; ether solvents such as di-*tert*-butylether, diethylether, diisopropyl ether, 1,4-dioxane, methyl *tert*-butylether, ethyl *tert*-butyl ether, tetrahydrofuran and dimethoxyethane; hydrocarbons solvents such as toluene, xylene, heptane, hexane and cyclohexane.

The hydrogenation of the present invention takes place in an alcoholic solvent.

Preferably, the hydrogenation takes place in the presence of methanol or isopropanol.

The term "acid" refers to an organic acid or an inorganic acid. Examples of acids that may be used include, but are not limited to, organic acids and/or inorganic acids, including carboxylic acids, monovalent, divalent or multivalent acids, linear or branched. Non-limiting examples of carboxylic acids include formic acid, acetic acid, propanoic acid, butanoic acid, pentanoic acid, hexanoic acid, heptanoic acid, octanoic acid, ascorbic acid, succinic acid, tartaric acid, lactic acid and the like or combinations of any two or more thereof. Particularly preferred carboxylic acids are acetic acid and octanoic acid. More preferably carboxylic acid is octanoic acid. The most preferable carboxylic acid is acetic acid.

In a preferred embodiment of the present invention, the carboxylic acid is selected from the group consisting of acetic acid and octanoic acid.

In a preferred embodiment of the present invention, the carboxylic acid is acetic acid.

Non-limiting examples of inorganic acids include one or more selected from hydrochloric acid and sulfuric acid.

Preferably, the amount of acid used is from 0.025 equivalents to 0.1 equivalents, more preferably from 0.04 equivalents to 0.07 equivalents, based on the 4-aminopyridine used in the preparation of the compound of formula II.

In a preferred embodiment of the present invention, the compound of formula II is prepared by a process comprising reacting 4-aminopyridine of formula III with an aldehyde of Formula IV 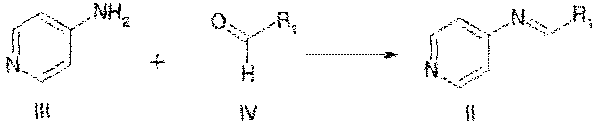 wherein R₁ is hydrogen or an alkyl having 1 to 11 carbon atoms.

In a preferred embodiment of the present invention, the alkyl is selected from the group consisting of methyl, ethyl, and linear or branched propyl, butyl, pentyl, hexyl, heptyl, octyl, nonyl, decyl and undecyl.

In a preferred embodiment of the present invention, the alkyl is n-heptyl.

The reaction of 4-aminopyridine with the aldehyde of formula IV takes place using an excess of the aldehyde. Preferably, the aldehyde is used in amounts of up to about 2 equivalents, preferably up to about 1.7 equivalents, based on the 4-aminopyridine of formula III.

The authors of the present invention have found that in the processes of the prior art it is not possible to achieve complete conversion of the 4-aminopyridine of formula III even in the presence of an excess of the aldehyde of formula IV. The presence of an acid in the process of the present invention allows the complete conversion of the 4-aminopyridine of formula III resulting in a *N*-alkyl-4-pyridinamine of formula I with high chemical purity which has been obtained through the catalytic hydrogenation of the corresponding compound of formula II.

The authors of the present invention have also found that the process of the invention allows to use lower amounts of catalyst than in the prior art which is desirable because of lower manufacturing cost.

Preferably, reaction of 4-aminopyridine of formula III with the aldehyde of formula IV is carried out in the presence of a solvent. Non-limiting examples of organic solvents, which can be used alone or as a mixture of solvents, include polar aprotic solvents such as dimethylsulfoxide, *N*,*N*-dimethylformamide, *N*,*N*-dimethylacetamide, *N,N-*dimethylformamide and the like; esters solvents such as butyl acetate, ethyl acetate, ethyl formate, isobutyl acetate, isopropyl acetate, methyl acetate, methyl formate, propyl acetate and the like; alcohol solvents such as methanol, ethanol, *n*-propanol, isopropanol, *n*-butanol and the like; ether solvents such as di-*tert*-butylether, diethylether, diisopropyl ether, 1,4-dioxane, methyl *tert*-butylether, ethyl *tert*-butyl ether, tetrahydrofuran and dimethoxyethane; hydrocarbons solvents such as toluene, xylene, heptane, hexane and cyclohexane. Preferably, the reaction takes place in the presence of alcohol solvents, more preferably in the presence of methanol or isopropanol.

Preferably, the reaction takes place at a temperature from about 10 °C to about 95 °C, more preferably from about 15 °C to about 70 °C, even more preferably from about 20 °C to about 25 °C.

The reaction of 4-aminopyridine with the aldehyde of formula IV to form the compound of formula II may be carried out by first dissolving the 4-aminopyridine in a solvent and then adding the aldehyde or by first dissolving the aldehyde in a solvent and then adding 4-aminopyridine. It may also be carried out by reacting 4-aminopyridine with the aldehyde in the absence of a solvent and, optionally, then adding a solvent.

The compound of formula II which is formed can be isolated prior to the catalytic hydrogenation or it can be hydrogenated in situ to obtain the *N*-alkyl-4-pyridinamine of formula I without isolation of the intermediate compound of formula II.

It is also possible to use the 4-aminopyridine, the aldehyde of formula IV and the solvent together with the catalyst all at once under hydrogen pressure to form *N*-alkyl-4-pyridinamine of formula I as long as compound of formula II is generated in situ and further hydrogenated.

Another aspect of the invention is that the process of the present invention further comprises the reaction of the *N*-alkyl-4-pyridinamine of formula I with a compound of formula V to prepare a bis(N-alkylpyridin-4(1 H)-imine of formula VI or a salt thereof. 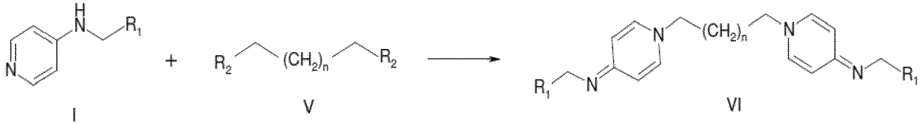 wherein R₁ is as defined above and R₂ is a leaving group and n is an integer from 0 to 8.

It is known to the person skilled in the art that bis(N-alkylpyridin-4(1H)-imine of formula VI can be in the form of salts, such as the hydrochloride salt.

The term "leaving group" refers to an electron-withdrawing atom or group of atoms activating the carbon to which they are attached against a nucleophilic reagent, such that said nucleophile, or part of said nucleophile, reacts with the molecule and the leaving group is detached therefrom. Preferably, the leaving group is Cl, Br, I, methanesulfonate (OMs), trifluoromethanesulfonate (OTf), p-toluenesulfonate (OTs) and benzenesulfonate (OBs). More preferably, the leaving group is Cl.

In a preferred embodiment of the present invention, R₂ is CI and n is 8, and consequently compound V is 1,10-dichlorodecane.

*N*-alkyl-4-pyridinamines of formula I are intermediates useful in the preparation of bis(N-alkylpyridin-4(1H)-imines which are compounds with bacteriostatic and bactericidal effects.

In a preferred embodiment of the present invention, the alkyl is selected from the group consisting of methyl, ethyl, and linear or branched propyl, butyl, pentyl, hexyl, heptyl, octyl, nonyl, decyl and undecyl.

In a preferred embodiment of the present invention, the compound of formula VI is octenidine wherein alkyl is *n*-heptyl and n is 8.

In a more preferred embodiment of the present invention, the compound of formula VI is octenidine hydrochloride.

Preferably, the molar ratio of the compound of formula V versus the compound of formula I is from about 0.4 to about 0.6, more preferably from about 0.45 to about 0.55, even more preferably about 0.5.

Preferably, the compound of formula V is added over a solution of the compound of formula I in a solvent or *vice versa.* More preferably, the compound of formula V is added over a solution of the compound of formula I in a solvent.

The reaction preferably takes place in the presence of an organic solvent and optionally, in the presence of water. Non-limiting examples of organic solvents, which can be used alone or as a mixture of solvents, include polar aprotic solvents such as dimethylsulfoxide, *N*,*N*-dimethylformamide, *N*,*N*-dimethylacetamide, *N,N-*dimethylformamide and the like; esters solvents such as n-butyl acetate, ethyl acetate, ethyl formate, isobutyl acetate, isopropyl acetate, methyl acetate, methyl formate, propyl acetate and the like; alcohol solvents such as methanol, ethanol, *n*-propanol, isopropanol, *n*-butanol and the like; ether solvents such as di-*tert*-butylether, diethylether, diisopropyl ether, 1 ,4-dioxane, methyl *tert*-butylether, ethyl *tert*-butyl ether, tetrahydrofuran and dimethoxyethane; hydrocarbon solvents such as toluene, xylene, heptane, hexane and cyclohexane and mixtures thereof. Preferably, the reaction takes place in the presence of ester solvents, more preferably in the presence of n-butyl acetate.

Preferably, the reaction between the compound of formula I and the compound of formula V is carried out under reflux temperature.

Another aspect of the invention is that the octenidine hydrochloride obtained according to the process of the present invention can be converted to octenidine base. The authors of the present invention have found that octenidine hydrochloride by a neutralization process can be converted to a crude of octenidine base and that the crude can be purified to obtain a crystalline octenidine base.

### Examples

### X-Ray Powder Diffraction (XRPD):

The XRPD pattern was recorded on a Siemens D5000 diffractometer equipped with two symmetrically mounted vertical goniometers (Bragg-Brentano geometry) with horizontal sample stages, an X-ray tube, a high voltage generator (working at 45 kV and 35 mA) and standard scintillation detectors. Ni-filtered Cu-anode source was used, and diffracted radiation was further monochromatized with a graphite crystal to avoid fluorescence effects [(λ(K_{α}) = 1.54056 Å]. The diffraction pattern was recorded including values of 2θ that range from 2 to 50° with a sampling rate of 0.02° per second and a step time of 1 second per step. The powdered sample was pressed between two glass plates, forming a film. DIFFRAC Plus measurement software with EVA evaluation software (Bruker) was used to record the data and for a primary analysis of the diffraction pattern.

The equipment was periodically calibrated using quartz and silicon.

### Comparative example 1: Preparation of N-octylpyridin-4-amine

52.5 g (0.558 mol) of 4-aminopyridine, 390 ml of ethanol, 148 ml (0.948 mol) of octanal and 3.9 g (dry weight, 7.4% w/w with regard to 4-aminopyridine) of 10% Pd/C were mixed at about 20-25 °C. The resulting mixture was heated to about 80 °C and stirred at this temperature for 13.5 hours under a hydrogen pressure of about 3.5 bar. Then, 26 ml of octanal were added and the reaction mixture was stirred for 7 hours while keeping the temperature at about 80 °C and the hydrogen pressure at about 3.5 bar. The control by TLC showed the presence of unreacted 4-aminopyridine. After decreasing the temperature to about 20-25 °C, the reaction mixture was filtered, and the solution was concentrated to dryness. The oily residue obtained was triturated with 230 ml of heptane at about 0 °C and the resulting suspension was filtered. The obtained wet solid was dried at about 60 °C under vacuum to give 85.3 g of *N*-octylpyridin-4-amine. Total yield: 74.1%, starting from 4-aminopyridine. Purity (% area, HPLC): 99.03%; 4-aminopyridine: 0.55%; Schiff base (compound II with R₁= *n*-heptyl): 0.19%.

### Comparative example 2: Preparation of N-octylpyridin-4-amine

70 g (0.744 mol) of 4-aminopyridine were dissolved in 480 ml of ethanol and 232 ml (1.486 mol) of octanal and stirred at about 80 °C for 1 hour. After cooling to about 20-25 °C, a mixture of 5.2 g (dry weight, 7.4% w/w with regard to 4-aminopyridine) of 10% Pd/C and 60 ml of ethanol were added thereto. The reaction mixture was heated to 80 °C and stirred for 8 hours under a hydrogen pressure of about 3.5 - 4.0 bar. Afterwards, 1.3 g (dry weight, 1.9% w/w with regard to 4-aminopyridine) of 10% Pd/C were added and the reaction mixture was stirred for 4 hours while keeping the temperature at about 80 °C and the hydrogen pressure at about 3.5 bar. The control by TLC showed the presence of unreacted 4-aminopyridine. After cooling to about 20-25 °C, the reaction mixture was filtered, and the solution was concentrated to dryness. 80 ml of heptane were added to the oily residue and the solution was concentrated to dryness. The residue was dissolved with 79 ml of heptane at reflux temperature, the solution was cooled to about 0 °C and the resulting suspension was filtered. The obtained wet solid was dried at about 40 °C under vacuum to give 129 g of *N*-octylpyridin-4-amine. Yield: 81.6%, starting from 4-aminopyridine. Purity (% area, HPLC): 98.88%; 4-aminopyridine: 0.13%; Schiff base (compound II with R₁= *n*-heptyl): 0.87%.

### Comparative example 3: Preparation of N-octylpyridin-4-amine

60 g (0.638 mol) of 4-aminopyridine were dissolved in 300 ml of methanol and 199 ml (1.274 mol) of octanal and stirred at about 80 °C for 1 hour. After cooling to about 20-25 °C, a mixture of 4.44 g (dry weight, 7.4% w/w with regard to 4-aminopyridine) of 10% Pd/C and 35 ml of methanol were added thereto. The reaction mixture was heated to 80 °C and stirred for 23 hours with a hydrogen pressure of about 5.0-5.5 bar. The control by TLC showed the presence of unreacted 4-aminopyridine. After cooling to about 20-25 °C, the reaction mixture was filtered, and the solution was concentrated to dryness. 65 ml of heptane were added to the oily residue and the solution was concentrated to dryness. The oily residue was dissolved with 130 ml of heptane at reflux temperature, then the solution was cooled to about 0 °C and the resulting suspension was filtered. The obtained wet solid was dried at about 40 °C under vacuum to give 104.4 g of *N-*octylpyridin-4-amine. Yield: 79.4%, starting from 4-aminopyridine. Purity (% area, HPLC): 94.1%; 4-aminopyridine: not detected; Schiff base (compound II with R₁= *n-*heptyl): 5.9%.

### EXAMPLE 1: Preparation of N-octylpyridin-4-amine (in presence of octanoic acid)

35 g (0.372 mol) of 4-aminopyridine, 260 ml of methanol, 116 ml (0.743 mol) of octanal, 2.6 g (dry weight, 7.4% w/w with regard to 4-aminopyridine) of 10% Pd/C and 3.83 ml (0.024 mol) of octanoic acid were mixed at about 20-25 °C. The reaction mixture was heated to 80°C and stirred at this temperature for 9.5 hours under a hydrogen pressure of about 4.0-5.0 bar. The control by TLC showed complete conversion of 4-aminopyridine. After cooling to about 20-25 °C, the reaction mixture was filtered, and the solution was concentrated to dryness. 35 ml of heptane were added to the oily residue and the solution was concentrated to dryness. The residue was dissolved with 77 ml of heptane at reflux temperature, the solution was cooled to about 0°C and the resulting suspension was filtered. The solid was dried at about 40°C under vacuum to give 64.0 g of *N*-octylpyridin-4-amine. Yield: 83.5%, starting from 4-aminopyridine. Purity (% area, HPLC): 99.97%. 4-aminopyridine: not detected; Schiff base (compound II with R₁= *n-*heptyl): not detected.

### EXAMPLE 2: Preparation of N-octylpyridin-4-amine (in presence of acetic acid)

45 g (0.478 mol) of 4-aminopyridine, 250 ml of isopropanol, 127 ml (0.813 mol) of octanal, 1.13 g (dry weight, 2.5% w/w with regard to 4-aminopyridine) of 10% Pd/C and 1.12 ml (0.020 mol) of glacial acetic acid were mixed at about 20-25 °C. The reaction mixture was heated to about 80°C and stirred at this temperature for 17.5 hours under a hydrogen pressure of about 4.0-5.0 bar. The control by TLC showed complete conversion of 4-aminopyridine. After cooling to about 20-25 °C, the reaction mixture was filtered, and the solution was concentrated to dryness. 100 ml of heptane were added to the oily residue and the solution was concentrated to dryness. The oily residue was dissolved with 99 ml of heptane at reflux temperature, then the solution was cooled to about 0°C and the resulting suspension was filtered. The solid was dried at about 40°C under vacuum to give 94.6 g of *N*-octylpyridin-4-amine. Yield: 95.9%, starting from 4-aminopyridine. Purity (% area, HPLC): 99.98%. 4-aminopyridine: not detected; Schiff base (compound II with R₁= *n*-heptyl): 0.02%.

### EXAMPLE 3: Preparation of N-octylpyridin-4-amine (in presence of acetic acid)

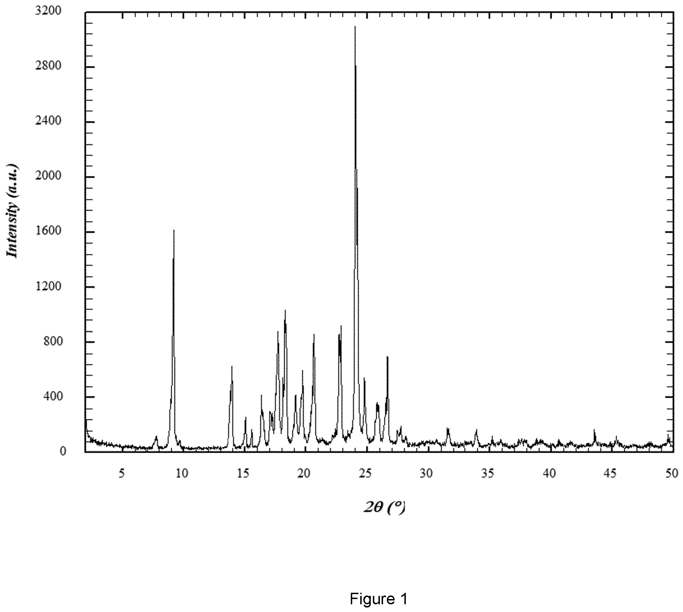

90 g (0.956 mol) of 4-aminopyridine were dissolved in 400 ml of methanol. 254 ml (1.627 mol) of octanal were added dropwise at about 20-25°C while stirring. Afterwards, 2.74 ml (0.048 mol) of glacial acetic acid, 3.15 g (dry weight, 3.5% w/w with regard to 4-aminopyridine) of 10% Pd/C and 100 ml of methanol were added to the reaction mixture. After heating to 80°C, the reaction was stirred at this temperature for 14 hours under a hydrogen pressure of about 4.0-5.0 bar. The control by TLC showed complete conversion of 4-aminopyridine. After cooling to about 20-25 °C, the reaction mixture was filtered, and the solution was concentrated to dryness. 99 ml of heptane were added to the oily residue and the solution was concentrated to dryness. The residue was dissolved with 197 ml of heptane at reflux temperature, then the solution was cooled to about 0°C and the resulting suspension was filtered. The obtained wet solid was dried at about 40°C under vacuum to give 166 g of *N*-octylpyridin-4-amine. Yield: 83.9%, starting from 4-aminopyridine. Purity (% area, HPLC): 99.95%. 4-aminopyridine: not detected; Schiff base (compound II with R₁= *n*-heptyl): 0.01%; XRPD: Diffraction peaks at (2θ, degrees): 9.1, 13.9, 17.7, 18.3, 20.6, 22.8, 24.0, 24.2 and 26.6 ± 0.2 (See Figure 1).

### EXAMPLE 4: Preparation of octenidine dihydrochloride

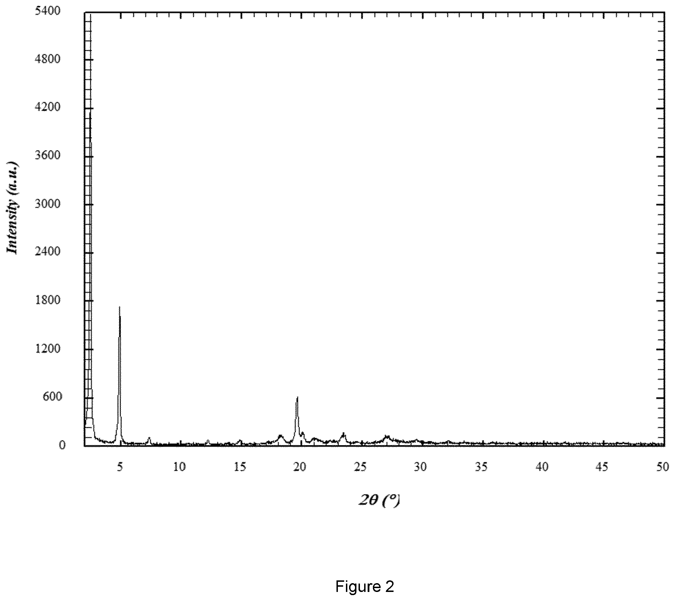

159.7 g (0.774 mol) of *N*-octylpyridin-4-amine obtained in example 3 were added to 240 ml of *n*-butyl acetate. The resulting suspension was heated to reflux temperature and 82.6 ml (0.387 mol) of 1,10-dichlorodecane were added dropwise. The reaction mixture was stirred for 12 hours at reflux temperature. After cooling to 40-50 °C, 966 ml of acetone and 241 ml of dimethylsulfoxide were added. The resulting suspension was heated to reflux temperature and water was added until complete dissolution. The solution was then cooled to 0-5°C and stirred at this temperature for 1 hour. After collecting by filtration the precipitated solid, 308 ml of acetone and then 53 ml of water were added. The resulting suspension was heated to reflux temperature and the solution obtained was filtered. Afterwards, 1129 ml of acetone were added onto the filtrate. After heating the resulting suspension to reflux temperature, water was added until complete dissolution. The solution was then cooled to 0-5°C and stirred at this temperature for 1 hour. The resulting suspension was filtered, obtaining a white solid which was dried at 45-50 °C under vacuum to give 201.2 g of octenidine dihydrochloride (Total yield: 85.1% based on *N*-octylpyridin-4-amine). Purity (% area, HPLC): 99.91%. XRPD: Diffraction peaks at (2θ, degrees): 2.5, 4.9, 18.3, 19.6, 20.1, 23.5, 26.9 and 27.1 ± 0.2 (See Figure 2).

### EXAMPLE 5: Preparation of octenidine dihydrochloride

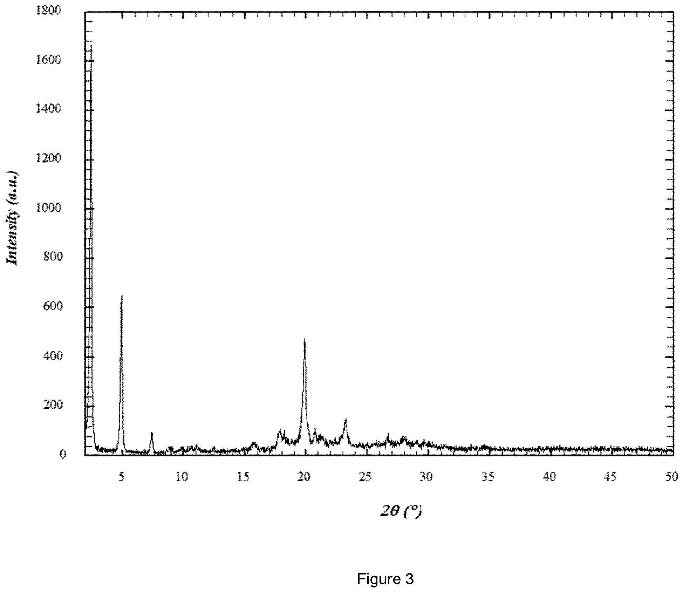

100 g (0.485 mol) of *N*-octylpyridin-4-amine obtained according to the process described in example 3 were added to 150 ml of n-butyl acetate. The resulting suspension was heated to reflux temperature and 51.1 ml (0.242 mol) of 1,10-dichlorodecane were added dropwise. The reaction mixture was stirred for 12 hours at reflux temperature. After cooling to 20-25°C, 605 ml of acetone and 151 ml of dimethyl sulfoxide were added. The resulting suspension was heated to reflux temperature and water was added until complete dissolution. The solution was then cooled to 0-5°C and stirred at this temperature for 1 hour. After collecting by filtration the precipitated solid, 193 ml of acetone and then 34 ml of water were added. The resulting suspension was heated to reflux temperature and the solution obtained was filtered. Afterwards, 706 ml of acetone were added onto the filtrate. After heating the resulting suspension to reflux temperature, water was added until complete dissolution. The solution was then cooled to 0-5°C and stirred at this temperature for 1 hour. The resulting suspension was filtered, obtaining a white solid which was dried at 45-50 °C under vacuum to give 122.5 g of octenidine dihydrochloride (Total yield: 81% based on *N*-octylpyridin-4-amine). Purity (% area, HPLC): 99.93%. XRPD: Diffraction peaks at (2θ, degrees): 2.4, 4.9, 17.9, 18.2, 19.9, 20.7 and 23.2 ± 0.2 (See Figure 3).

### EXAMPLE 6: Preparation of octenidine base

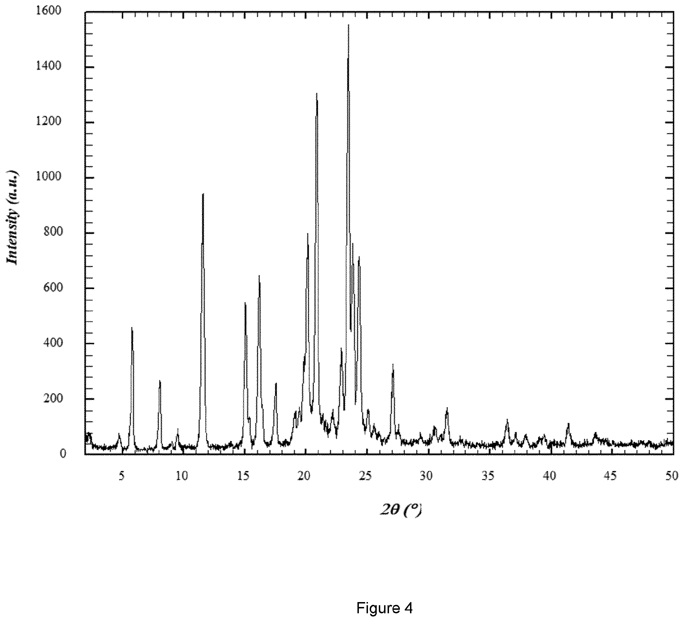

30 g of octenidine dihydrochloride obtained in example 4, 300 ml of toluene, 240 ml of 2N aqueous NaOH and 540 ml of deionized water were stirred for 1 hour at 20-25°C. The suspension was filtered obtaining a solid. The biphasic mother liquors were allowed to settle, and the aqueous phase was separated. The aqueous phase and the solid were combined together with 200 ml of toluene, 50 ml of 2N aqueous NaOH and 2 ml of 50% aqueous NaOH and the suspension was stirred at 30°C. The solution obtained was allowed to settle and a tri-phasic mixture was obtained. The upper organic phase was separated, washed with 100 ml of 1N aqueous NaOH at 20-25°C and the mixture was allowed to settle. A tri-phasic mixture was obtained. The upper organic phase was separated, washed again with 100 ml of 1N aqueous NaOH at 20-25°C and the mixture was allowed to settle. A tri-phasic mixture was obtained. The upper organic phase was separated, washed again with 100 ml of 1N aqueous NaOH at 30°C and the mixture was allowed to settle. A bi-phasic mixture was obtained, and the aqueous phase was discarded. The solvent from the organic phase was removed by distillation at reduced pressure at 50°C. The crude thus obtained was triturated with 60 ml of toluene at 50°C for 45 minutes, the suspension was stirred at 20-25°C for about 1 hour and then it was filtered, washing the cake with 5 ml of ethyl acetate. 14.5 g of octenidine base were obtained after drying the solid. Chloride content: 0.05%. Purity (% area, HPLC): 99.9%. XRPD: Diffraction peaks at (2θ, degrees): 5.8, 8.0, 11.6, 15.1, 16.1, 20.1, 20.8, 23.4, 23.8 and 24.3 ± 0.2 (See Figure 4).

## Claims

1. A process for preparing a *N*-alkyl-4-pyridinamine of formula I, 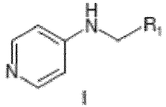
wherein R₁ is hydrogen or an alkyl having 1 to 11 carbon atoms,
which comprises the catalytic hydrogenation of the compound of formula II 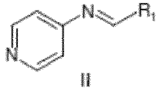
**characterized in that** the hydrogenation process is carried out in presence of an acid, a palladium catalyst, and an alcoholic solvent.

2. The process according to claim 1, wherein the alkyl is selected from the group consisting of methyl, ethyl, and linear or branched propyl, butyl, pentyl, hexyl, heptyl, octyl, nonyl, decyl and undecyl.

3. The process according to claim 2, wherein the alkyl is n-heptyl.

4. The process according to any of claims 1 to 3, wherein the alcoholic solvent is selected from methanol and isopropanol.

5. The process according to claim 4, wherein the catalyst is palladium-on-carbon (Pd/C).

6. The process according to any of claims 1 to 5, wherein the compound of formula II is prepared by a process comprising reacting 4-aminopyridine of formula III with an aldehyde of Formula IV 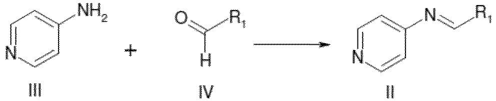 wherein R₁ is hydrogen or an alkyl having 1 to 11 carbon atoms.

7. The process according to claim 6, wherein the alkyl is selected from the group consisting of methyl, ethyl, and linear or branched propyl, butyl, pentyl, hexyl, heptyl, octyl, nonyl, decyl and undecyl.

8. The process according to claim 7, wherein the alkyl is n-heptyl.

9. The process according to any of claims 1 to 8, wherein the N-alkyl-4-pyridinamine of formula I is obtained without isolation of the intermediate compound of formula II.

10. The process according to any of claims 1 to 9, which further comprises the reaction of the *N*-alkyl-4-pyridinamine of formula I with a compound of formula V to prepare a bis(N-alkylpyridin-4(1H)-imine of formula VI or a salt thereof 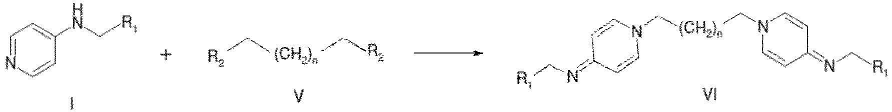 wherein R₁ is as defined above and R₂ is a leaving group and n is an integer from 0 to 8.

11. The process according to claim 10, wherein the alkyl is selected from the group consisting of methyl, ethyl, and linear or branched propyl, butyl, pentyl, hexyl, heptyl, octyl, nonyl, decyl and undecyl.

12. The process according to claim 11, wherein the alkyl is n-heptyl, n is 8 and R₂ is Cl.

13. The process according to claim 12, wherein the compound of formula VI is octenidine wherein alkyl is *n*-heptyl and n is 8 or a salt thereof.

14. The process according to claim 13, wherein the compound of formula VI is octenidine hydrochloride.

15. The process according to claim 14, wherein the octenidine hydrochloride is converted to octenidine base.

## Patentansprüche

1. Ein Verfahren zur Herstellung eines *N*-Alkyl-4-pyridinamins der Formel I, 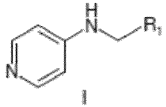
wobei R₁ Wasserstoff oder ein Alkyl mit 1 bis 11 Kohlenstoffatomen ist,
welches die katalytische Hydrierung der Verbindung der Formel II umfasst 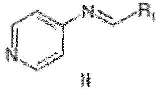
**dadurch gekennzeichnet, dass** das Hydrierungsverfahren in Gegenwart einer Säure, eines Palladiumkatalysators und eines alkoholischen Lösungsmittels durchgeführt wird.

2. Das Verfahren nach Anspruch 1, wobei das Alkyl ausgewählt ist aus der Gruppe bestehend aus Methyl, Ethyl und linearem oder verzweigtem Propyl, Butyl, Pentyl, Hexyl, Heptyl, Octyl, Nonyl, Decyl und Undecyl.

3. Das Verfahren nach Anspruch 2, wobei das Alkyl n-Heptyl ist.

4. Das Verfahren nach einem der Ansprüche 1 bis 3, wobei das alkoholische Lösungsmittel ausgewählt ist aus Methanol und Isopropanol.

5. Das Verfahren nach Anspruch 4, wobei der Katalysator Palladium auf Kohle (Pd/C) ist.

6. Das Verfahren nach einem der Ansprüche 1 bis 5, wobei die Verbindung der Formel II durch ein Verfahren hergestellt wird, das die Umsetzung von 4-Aminopyridin der Formel III mit einem Aldehyd der Formel IV umfasst, 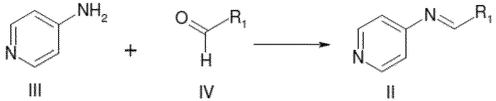 wobei R₁ Wasserstoff oder ein Alkyl mit 1 bis 11 Kohlenstoffatomen ist.

7. Das Verfahren nach Anspruch 6, wobei das Alkyl ausgewählt ist aus der Gruppe bestehend aus Methyl, Ethyl und linearem oder verzweigtem Propyl, Butyl, Pentyl, Hexyl, Heptyl, Octyl, Nonyl, Decyl und Undecyl.

8. Das Verfahren nach Anspruch 7, wobei das Alkyl *n*-Heptyl ist.

9. Das Verfahren nach einem der Ansprüche 1 bis 8, wobei das N-Alkyl-4-pyridinamin der Formel I ohne Isolierung der Zwischenverbindung der Formel II erhalten wird.

10. Das Verfahren nach einem der Ansprüche 1 bis 9, welches ferner die Umsetzung des N-Alkyl-4-pyridinamins der Formel I mit einer Verbindung der Formel V umfasst, um ein Bis(N-Alkylpyridin-4(1H)-imin der Formel VI oder ein Salz davon herzustellen 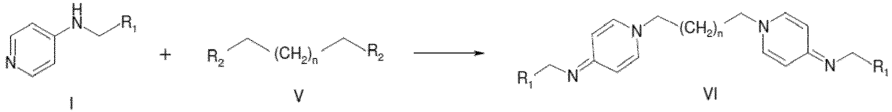 wobei R₁ wie oben definiert ist und R₂ eine Abgangsgruppe ist und n eine ganze Zahl von 0 bis 8 ist.

11. Das Verfahren nach Anspruch 10, wobei das Alkyl ausgewählt ist aus der Gruppe bestehend aus Methyl, Ethyl und linearem oder verzweigtem Propyl, Butyl, Pentyl, Hexyl, Heptyl, Octyl, Nonyl, Decyl und Undecyl.

12. Das Verfahren nach Anspruch 11, wobei das Alkyl *n*-Heptyl ist, n 8 ist und R₂ CI ist.

13. Das Verfahren nach Anspruch 12, wobei die Verbindung der Formel VI Octenidin ist, wobei das Alkyl *n*-Heptyl ist und n 8 ist, oder ein Salz davon.

14. Das Verfahren nach Anspruch 13, wobei die Verbindung der Formel VI Octenidinhydrochlorid ist.

15. Das Verfahren nach Anspruch 14, wobei das Octenidinhydrochlorid in Octenidinbase umgewandelt wird.

## Revendications

1. Un procédé de préparation d'une *N*-alkyl-4-pyridinamine de formule I, 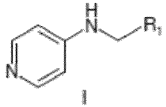
dans laquelle R₁ est hydrogène ou un alkyle ayant de 1 à 11 atomes de carbone,
qui comprend l'hydrogénation catalytique du composé de formule Il 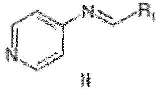
**caractérisé en ce que** le procédé d'hydrogénation est effectué en présence d'un acide, d'un catalyseur au palladium et d'un solvant alcoolique.

2. Le procédé selon la revendication 1, dans lequel l'alkyle est choisi dans le groupe constitué par le méthyle, l'éthyle et le propyle, le butyle, le pentyle, l'hexyle, l'heptyle, l'octyle, le nonyle, le décyle et l'undécyle linéaires ou ramifiés.

3. Le procédé selon la revendication 2, dans lequel l'alkyle est le n-heptyle.

4. Le procédé selon l'une quelconque des revendications 1 à 3, dans lequel le solvant alcoolique est choisi parmi le méthanol et l'isopropanol.

5. Le procédé selon la revendication 4, dans lequel le catalyseur est du palladium sur charbon (Pd/C).

6. Le procédé selon l'une quelconque des revendications 1 à 5, dans lequel le composé de formule Il est préparé par un procédé comprenant la réaction de la 4-aminopyridine de formule III avec un aldéhyde de formule IV, 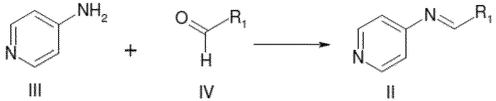 dans laquelle R₁ est hydrogène ou un alkyle ayant de 1 à 11 atomes de carbone,

7. Le procédé selon la revendication 6, dans lequel l'alkyle est choisi dans le groupe constitué par le méthyle, l'éthyle et le propyle, le butyle, le pentyle, l'hexyle, l'heptyle, l'octyle, le nonyle, le décyle et l'undécyle linéaires ou ramifiés.

8. Le procédé selon la revendication 7, dans lequel l'alkyle est le *n*-heptyle.

9. Le procédé selon l'une quelconque des revendications 1 à 8, dans lequel la N-alkyl-4-pyridinamine de formule I est obtenue sans isolement du composé intermédiaire de formule II.

10. Le procédé selon l'une quelconque des revendications 1 à 9, qui comprend en outre la réaction de la *N*-alkyl-4-pyridinamine de formule I avec un composé de formule V pour préparer une bis(N-alkylpyridine-4(1H)-imine de formule VI ou un sel de celle-ci, 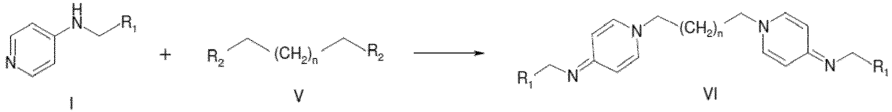 dans laquelle R₁ est tel que défini ci-dessus et R₂ est un groupe partant et n est un nombre entier allant de 0 à 8.

11. Le procédé selon la revendication 10, dans lequel l'alkyle est choisi dans le groupe constitué par le méthyle, l'éthyle et le propyle, le butyle, le pentyle, l'hexyle, l'heptyle, l'octyle, le nonyle, le décyle et l'undécyle linéaires ou ramifiés.

12. Le procédé selon la revendication 11, dans lequel l'alkyle est le *n*-heptyle, n est 8 et R₂ est Cl.

13. Le procédé selon la revendication 12, dans lequel le composé de formule VI est l'octénidine dans laquelle l'alkyle est le *n*-heptyle et n est 8 ou un sel de celui-ci.

14. Le procédé selon la revendication 13, dans lequel le composé de formule VI est le chlorhydrate d'octénidine.

15. Le procédé selon la revendication 14, dans lequel le chlorhydrate d'octénidine est converti en octénidine base.
